(19) Europäisches Patentamt
European Patent Office
Office européen des brevets

(11) **EP 4 455 311 A1**

(12) **EUROPEAN PATENT APPLICATION**
published in accordance with Art. 153(4) EPC

(43) Date of publication:
**30.10.2024 Bulletin 2024/44**

(21) Application number: **22928862.6**

(22) Date of filing: **28.10.2022**

(51) International Patent Classification (IPC):
*C21B 5/00* (2006.01)    *C07C 29/151* (2006.01)
*C21B 5/06* (2006.01)    *C21B 7/00* (2006.01)
*F27D 17/00* (2006.01)

(52) Cooperative Patent Classification (CPC):
**C07C 29/151; C21B 5/00; C21B 5/06; C21B 7/00; F27D 17/00**

(86) International application number:
**PCT/JP2022/040553**

(87) International publication number:
**WO 2023/162344 (31.08.2023 Gazette 2023/35)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **24.02.2022 JP 2022027239**

(71) Applicant: **JFE Steel Corporation
Tokyo 100-0011 (JP)**

(72) Inventors:
• **UCHIDA, Seiji**
**Tokyo 100-0011 (JP)**
• **TAKAHASHI, Koichi**
**Tokyo 100-0011 (JP)**
• **KAWASHIRI, Yuki**
**Tokyo 100-0011 (JP)**
• **KASHIHARA, Yusuke**
**Tokyo 100-0011 (JP)**

(74) Representative: **Hoffmann Eitle
Patent- und Rechtsanwälte PartmbB
Arabellastraße 30
81925 München (DE)**

(54) **METHOD FOR OPERATING BLAST FURNACE, METHOD FOR PRODUCING HOT METAL, AND AUXILIARY FACILITY FOR BLAST FURNACE**

(57)    Provided is a method of operating a blast furnace that can further reduce carbon dioxide emissions from the blast furnace under stable operation. A blown reducing agent into the blast furnace contains a regenerative organic substance and the regenerative organic substance is generated by using at least one of carbon monoxide or carbon dioxide in the blast furnace by-product gas discharged from the blast furnace with hydrogen and has a specific energy Q of 4000 kJ/kg or more.

*FIG. 1*

EP 4 455 311 A1

**Description**

TECHNICAL FIELD

This disclosure relates to a method of operating a blast furnace, a method of producing hot metal, and a blast furnace ancillary facility.

BACKGROUND

In recent years, there has been a strong need to reduce carbon dioxide ($CO_2$) emissions against the backdrop of global environmental issues. Therefore, operation with a low reducing agent rate (low RAR) is required in operating a blast furnace installed in a steelworks.

In a typical blast furnace, hot blast (air heated to about 1200 °C) is blown into the blast furnace as a blast gas through a tuyere. As a result, oxygen in the hot blast reacts with coke and pulverized coal as reducing agents to generate gaseous carbon monoxide (CO) and hydrogen ($H_2$). Such carbon monoxide and hydrogen reduce iron ore charged into the blast furnace. In addition, carbon dioxide is generated during the reduction reaction of the iron ore.

The following technologies have been proposed to reduce carbon dioxide emissions in such operation of the blast furnace. In detail, carbon monoxide and carbon dioxide contained in the by-product gas discharged from the blast furnace (hereinafter referred to as blast furnace by-product gas) are reformed to generate compounds such as methane and ethanol. These compounds are then reintroduced into the blast furnace as reducing agents.

For example, JP2011-225969A (PTL 1) describes:
"a method of operating a blast furnace comprising a step (A) of separating and recovering $CO_2$ and/or CO from a mixed gas containing $CO_2$ and/or CO, a step (B) of adding hydrogen to the $CO_2$ and/or CO separated and recovered in the step (A) and converting the $CO_2$ and/or CO into $CH_4$, a step (C) of separating and removing $H_2O$ from the gas that has undergone the step (B), and a step (D) of blowing the gas that has undergone the step (C) into the blast furnace."

JP2014-005510A (PTL 2) describes:
"a method of operating a blast furnace comprising separating $CO_2$ from an exhaust gas of a combustion furnace that uses a blast furnace by-product gas as the whole or part of fuel, reforming the separated $CO_2$ into methane to obtain a reducing gas, and blowing the reducing gas into the blast furnace."

CITATION LIST

Patent Literature

> PTL 1: JP 2011-225969A
> PTL 2: JP 2014-005510A

SUMMARY

(Technical Problem)

However, with the techniques of PTLs 1 and 2, when the amount of methane blown into the blast furnace as the reducing agent exceeds a certain level, it may cause operational problems such as insufficient heating of the bottom of the blast furnace, pressure drop increase, and tapping failure. Therefore, there is a need to limit the blowing amount of methane to achieve stable operation, and then the effectiveness of carbon dioxide emission reductions is limited.

It could thus be helpful to provide a method of operating a blast furnace that can further reduce carbon dioxide emissions from the blast furnace under stable operation.

It is also an object of this disclosure to provide a method of producing hot metal using the above-mentioned method of operating a blast furnace, and a blast furnace ancillary facility for the above-mentioned method of operating a blast furnace.

(Solution to Problem)

We therefore made intensive studies to achieve the objects stated above. As a result, we found that carbon monoxide and carbon dioxide contained in the blast furnace by-product gas are used with hydrogen to synthesize a regenerative organic substance with a large heating value during combustion, which can be used as a blown reducing agent to thereby greatly reduce carbon dioxide emissions. This disclosure was completed based on this finding, and primary features thereof are as described below.

1. A method of operating a blast furnace, comprising

blowing a blast gas and a blown reducing agent into the blast furnace through a tuyere,
wherein the blown reducing agent contains a regenerative organic substance,
the regenerative organic substance is generated using at least one of carbon monoxide or carbon dioxide in a blast furnace by-product gas, which is a by-product gas discharged from the blast furnace, with hydrogen, and
the regenerative organic substance has a specific energy Q of 4000 kJ/kg or more when converted to CO and $H_2$ by combustion reaction.

2. The method of operating a blast furnace according to 1., wherein $x_c + x_H$, a sum of a mass ratio $x_c$ of carbon atoms in the regenerative organic substance and a mass ratio $x_H$ of hydrogen atoms in the regenerative organic substance, is 90 wt% or more.
3. The method of operating a blast furnace according to 1. or 2., wherein the mass ratio $x_c$ of carbon atoms in the regenerative organic substance is 85 wt% or more.
4. The method of operating a blast furnace according to any of 1. to 3., wherein the regenerative organic substance contains at least one selected from the group consisting of ethylene, propylene, solid carbon, ethane, propane, and phenol.
5. The method of operating a blast furnace according to any of 1. to 4., wherein the regenerative organic substance contains 90 wt% or more in total of at least one of ethylene or propylene.
6. The method of operating a blast furnace according to any of 1. to 5., wherein the blown reducing agent contains 80 wt% or more of the regenerative organic substance.
7. The method of operating a blast furnace according to any of 1. to 6., wherein the regenerative organic substance is generated from part of the blast furnace by-product gas and a surplus of the blast furnace by-product gas is supplied inside or outside a steelworks.
8. The method of operating a blast furnace according to any of 1. to 7., wherein a surplus of the regenerative organic substance is supplied inside or outside the steelworks.
9. A method of producing hot metal using the method of operating a blast furnace according to any of 1. to 8.
10. A blast furnace ancillary facility used in the method of operating a blast furnace according to any of 1. to 8., comprising:

an organic substance generation device that uses the blast furnace by-product gas to generate the regenerative organic substance, and
a gas and reducing agent blowing device having a blown reducing agent supply section that introduces the blown reducing agent into the tuyere of the blast furnace and a blast gas supply section that introduces the blast gas into the tuyere of the blast furnace.

(Advantageous Effect)

This disclosure can provide a method of operating a blast furnace that can further reduce carbon dioxide emissions from the blast furnace under stable operation. Also, can be provided a method of producing hot metal using the method of operating a blast furnace, and a blast furnace ancillary facility used for the method of operating a blast furnace.

BRIEF DESCRIPTION OF THE DRAWINGS

In the accompanying drawings:

FIG. 1 schematically illustrates an example of a blast furnace and blast furnace ancillary facility used in the method of operating a blast furnace according to one of the disclosed embodiments;
FIGS. 2A and 2B each schematically illustrate an example of a gas and reducing agent blowing device used in the method of operating a blast furnace according to one of the disclosed embodiments;
FIG. 3 schematically illustrates an example of a blast furnace and blast furnace ancillary facility used in a comparative example;
FIG. 4 schematically illustrates an example of a blast furnace and blast furnace ancillary facility used in the method of operating a blast furnace according to one of the disclosed embodiments; and

FIG. 5 schematically illustrates an example of a blast furnace and blast furnace ancillary facility used in a comparative example.

DETAILED DESCRIPTION

The following describes the embodiments of this disclosure. This disclosure is not limited to the following embodiments. In the present specification, a numerical range expressed by using "to" means a range including numerical values described before and after "to", as the lower and upper limit values.

The method of operating a blast furnace according to one of the disclosed embodiments comprises blowing a blast gas and a blown reducing agent into the blast furnace through a tuyere,

wherein the blown reducing agent contains a regenerative organic substance,
the regenerative organic substance is generated using at least one of carbon monoxide or carbon dioxide in a blast furnace by-product gas, which is a by-product gas discharged from the blast furnace, with hydrogen, and
the regenerative organic substance has a heating value Q of 4000 kJ/kg or more when converted to CO and $H_2$ by combustion reaction.

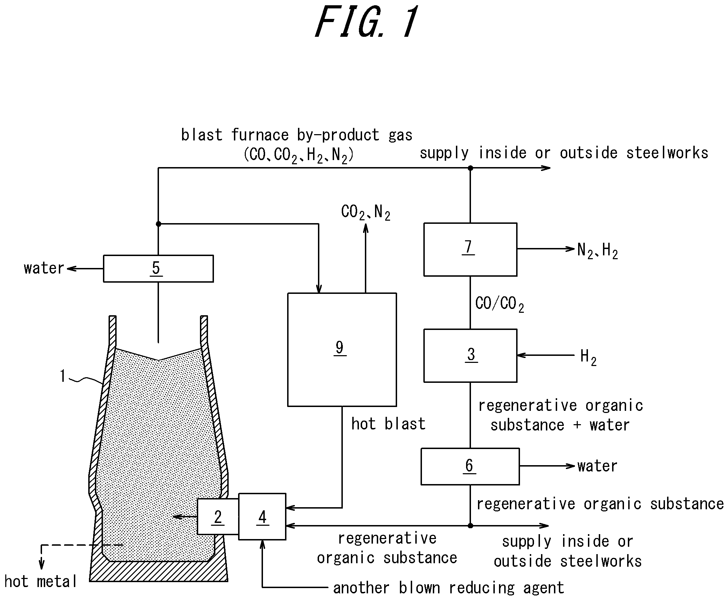

First, the method of operating a blast furnace according to one of the disclosed embodiments will be described, taking as an example the case where the method is applied to the blast furnace and blast furnace ancillary facility schematically illustrated in FIG. 1. In the figure, reference sign 1 is a blast furnace, 2 is a tuyere, 3 is an organic substance generation device, 4 is a gas and reducing agent blowing device, 5 is a first dehydration device, 6 is a second dehydration device, 7 is a gas separation device, and 9 is a hot air oven.

As used herein, the term "blast furnace" includes a shaft-type reducing furnace.

[How to operate a blast furnace]

In the method of operating a blast furnace according to one of the disclosed embodiments, sintered ore, lump ore, and pellet, which are raw materials (hereinafter also referred to as ore materials), coke, and the like are charged into the blast furnace from the top (not illustrated). In addition, the blast gas and reducing agent are blown into the blast furnace 1 through the tuyere 2 provided at the bottom of the blast furnace. The reducing agent that is blown into the blast furnace 1 through the tuyere 2 is also called "blown reducing agent" to distinguish it from coke. The blast gas, which is a gas blown into the blast furnace through the tuyere, also plays a role in gasifying the pulverized coal and coke in the blast furnace.

The gaseous carbon monoxide and hydrogen generated by the reaction between the blast gas and the blown reducing agent reduce the ore material charged into the blast furnace 1 to produce hot metal. In this reduction reaction of the ore material, carbon dioxide is generated and discharged from the top of the blast furnace as a by-product gas, along with carbon monoxide and hydrogen that have not reacted with the ore material. The by-product gas discharged from the top of the blast furnace is the blast furnace by-product gas. The top of the blast furnace is under high pressure conditions of about 2.5 atm. Therefore, water vapor contained in the blast furnace by-product gas condenses due to the expansion and cooling of the blast furnace by-product gas as it returns to normal pressure. The condensate is then removed in the first dehydration device 5.

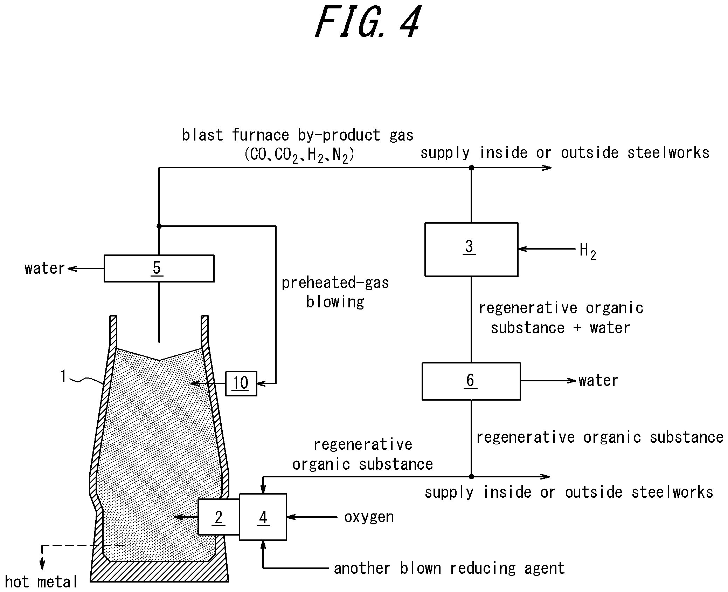

Next, at least part of the blast furnace by-product gas is preferably introduced into the gas separation device 7. Carbon monoxide and carbon dioxide are then separated from the blast furnace by-product gas in the gas separation device 7. For example, when oxygen gas is used as the blast gas, the blast furnace by-product gas may be introduced as it is into the organic substance generation device 3 without separating carbon monoxide and carbon dioxide from the blast furnace by-product gas, as illustrated in FIG. 4.

The carbon monoxide and carbon dioxide separated from the blast furnace by-product gas are then introduced into the organic substance generation device 3. Then, at least one of the carbon monoxide or carbon dioxide reacts with hydrogen in the organic substance generation device 3 to generate a regenerative organic substance. The regenerative organic substance here refers to a compound represented by the composition formula $C_nH_mO_l$ (n is an integer greater than or equal to 1, and m and l are integers greater than or equal to 0), which also includes solid carbon. The regenerative organic substance need not be pure material but may be a mixture of two or more compounds.

The method of generating the regenerative organic substance from at least one of carbon monoxide or carbon dioxide with hydrogen in the organic substance generation device 3 is not particularly limited, and an appropriate method should be selected according to the compound to be generated. For example, if the regenerative organic substance contains ethane or propane, the Fischer-Tropsch reaction can be used to synthesize the regenerative organic substance. If the regenerative organic substance contains ethylene or propylene, a synthesis method called the Methanol to Olefin (MTO) process or Methanol to Propylene (MTP) process can be used in addition to the Fischer-Tropsch reaction. In the synthesis method, methanol is synthesized by the reaction of at least one of carbon monoxide or carbon dioxide with hydrogen, and then methanol is converted to ethylene or propylene using a catalyst. If the regenerative organic substance contains solid carbon, a method such as synthesizing methane by the reaction of carbon monoxide and carbon dioxide with hydrogen and then pyrolyzing the synthesized methane to obtain solid carbon can be used.

The method of supplying a hydrogen-containing gas (hereinafter referred to as "hydrogen gas") used to generate the regenerative organic substance is not particularly limited, and the hydrogen gas can be supplied, for example, from within the steelworks or from outside the steelworks (hereinafter simply referred to as "outside"). Supply sources in the steelworks include, for example, by-product gas discharged from a coke oven (hereinafter referred to as "coke oven by-product gas"). When the hydrogen gas is produced in the steelworks, a method that generates as little carbon dioxide as possible is preferable. For example, electrolysis of water can be used. In addition, the hydrogen gas to be supplied from outside includes, for example, hydrogen gas produced by reforming hydrocarbons such as natural gas by steam reforming, etc., hydrogen gas obtained by vaporizing liquefied hydrogen, and hydrogen gas produced by dehydrogenating organic hydrides.

The hydrogen gas used to generate the regenerated organic substance may or may not have a hydrogen concentration: 100 % by volume. However, in order to increase the purity of the regenerative organic substance, a gas with a high hydrogen concentration, specifically, a hydrogen gas with a hydrogen concentration of 80 % or more by volume is preferred. The hydrogen concentration of the hydrogen gas is more preferably 90 % or more by volume, and further preferably 95 % or more by volume. The remainder gas other than hydrogen in the hydrogen gas includes, for example, CO, $CO_2$, $H_2S$, $CH_4$, and $N_2$.

Then, water in the regenerative organic substance is removed in the second dehydration device 6. The method for removing water is not particularly limited but includes for example, one that utilizes the difference in condensation points between water and regenerative organic substance and one that utilizes phase separation between water and regenerative organic substance.

As described above, at least one of carbon monoxide or carbon dioxide in the blast furnace by-product gas is used with hydrogen to generate the regenerative organic substance. The gas separation device 7 and organic substance generation device 3 do not necessarily need to be located adjacent to the blast furnace, nor do they need to be in the steelworks. For example, the blast furnace by-product gas is provided outside, and at least one of carbon monoxide or carbon dioxide is separated from the blast furnace by-product gas in the gas separation device 7 being outside. Then, at least one of the carbon monoxide or carbon dioxide may be used with hydrogen in the organic substance generation device 3 being outside to generate the regenerative organic substance. This externally generated regenerative organic substance can also be used as a blown reducing agent. And in this disclosure, the regenerative organic substance is characterized in that it has a heating value Q (hereinafter also referred to as heating value Q of regenerative organic substance) of 4000 kJ/kg or more when converted to CO and $H_2$ by combustion reaction. The heating value Q of regenerative organic substance is the heating value when the regenerative organic substance blown in as a reducing agent through the tuyere undergoes a combustion reaction with the oxygen in the blast gas at the tuyere outlet of blast furnace to convert to CO and $H_2$.

When the regenerative organic substance generated from the blast furnace by-product gas is blown in through the tuyere as a blown reducing agent, as the blowing amount of regenerative organic substance increases, the amount of coke required is reduced, thereby reducing carbon dioxide emissions. However, as the blowing amount of regenerative organic substance is increased, the tuyere-outlet temperature decreases, making stable operation of the blast furnace difficult. In detail, there is an upper limit to the blowing amount of regenerated organic substance. For this, we focused on the fact that the regenerative organic substance blown in through the tuyere undergoes a combustion reaction with $O_2$ at the tuyere outlet, converting to CO and $H_2$, and found that the heating value Q, which is the heating value during the combustion reaction, has a significant effect on the tuyere-outlet temperature. Further, we found that if a regenerative organic substance with a heating value Q of 4000 kJ/kg or more is used as a blown reducing agent, it is possible to increase the blowing amount of blown reducing agent while maintaining a high temperature at the tuyere outlet.

Furthermore, by setting the heating value Q of regenerative organic substance to 4000 kJ/kg or more, the amount of heat supplied to the blast furnace by the combustion of regenerative organic substance becomes larger. This reduces the amount of coke used to supply heat. These two effects can significantly reduce carbon dioxide emissions from blast furnace operations.

The heating value Q of regenerative organic substance can be determined, for example, by a formula. The heating value Q of regenerative organic substance is defined, for example, by the formula (1) below. The heating value Q of regenerative organic substance in the examples described below was obtained by the following formula (1):

$$Q = Q_L\text{-}283000 \times (x_C/100/12) - 241800 \times (x_H/100/2) \qquad (1).$$

In the formula (1),

$Q_L$ is the lower heating value [kJ/kg] of regenerative organic substance,
xc is the mass ratio [wt%] of carbon atoms in the regenerative organic substance, and
$x_H$ is the mass ratio [wt%] of hydrogen atoms in the regenerative organic substance.

The lower heating value (net heating (calorific) value or net specific energy) $Q_L$ of regenerative organic substance can be measured by the method described in JIS K 2279.

The heating value Q of regenerative organic substance can also be obtained by measuring the heating value (heat of decomposition) when the regenerative organic substance decomposes to a single unit and subtracting the heat of decomposition from the heat of reaction when CO is formed from C and $O_2$.

Further, the regenerative organic substance may be blown into the coke-packed layer with $O_2$ and combusted to measure the temperature change and coke consumption during the combustion, and these measurements may be used to determine the heating value Q of regenerative organic substance. Specifically, it is determined as follows. First, the amount of heat received by the coke is determined from the mass of coke remaining unconsumed, coke specific heat, and temperature change, and the amount of heat carried out by the gas is determined from the gas temperature, gas specific heat, and gas flow rate at the outlet of the coke-packed layer. The sum of these amounts of heat is the total amount of heat generated during combustion. Next, the amount of heat generated by coke combustion is determined from the amount of coke consumed and heat of coke combustion. The heating value Q of regenerative organic substance is then determined by excluding the amount of heat generated by coke combustion from the total amount of heat generated during combustion.

When the regenerative organic substance contains two or more compounds, the heating value Q of regenerative organic substance can be determined by determining the heating value of each of the compounds constituting the regenerative organic substance and taking their mass average.

If the heating value Q of regenerative organic substance is less than 4000 kJ/kg, the tuyere-outlet temperature will decrease significantly when the blowing amount of regenerative organic substance is increased, increasing the risk of operational problems. Therefore, the blowing amount of regenerative organic substance cannot be increased, and carbon dioxide emissions cannot be significantly reduced. In addition, since the amount of heat supplied to the blast furnace is decreased, extra coke must be used for that amount of heat, resulting in a smaller reduction in carbon dioxide emissions. To more effectively reduce carbon dioxide emissions, the heating value Q of regenerative organic substance is preferably 6000 kJ/kg or more. No upper limit is particularly placed on the heating value Q of regenerative organic substance but 18000 kJ/kg or less may be preferable.

The derivation of formula (1) is explained below. Assuming that the average composition of regenerative organic substance is $C_nH_mO_l$ (n is an integer greater than or equal to 1, and m and l are integers greater than or equal to 0) and the average molar mass of regenerative organic substance is M [kg/mol], the combustion reaction at the tuyere outlet is expressed by the following formula (2):

$$C_nH_mO_l + (n/2\text{-}l/2)O_2 = nCO + (m/2)H_2 + QM \qquad (2).$$

From the definition of lower heating value,

$$C_nH_mO_l + (n + m/4\text{-}l/2)O_2 = nCO_2 + (m/2)H_2O + Q_LM \qquad (3).$$

The generating reactions of $CO_2$ and $H_2O$ can be expressed as follows:

$$CO + 1/2O_2 = CO_2 + 283.0 \text{ kJ/mol} \qquad (4);$$

and

$$H_2 + 1/2O_2 = H_2O + 241.8 \text{ kJ/mol} \qquad (5).$$

From the formulas (3) through (5), the following formula (6) is obtained:

$C_nH_mO_l + (n/2\text{-}1/2)O_2 = nCO + (m/2)H_2 + (Q_LM - 283.0 \times n - 241.8 \times m/2)kJ/mol$

Comparing the formulas (2) and (6), the following is obtained:

$$Q = Q_L - 283.0 \times n/M - 241.8 \times (m/2M) \qquad (7).$$

Since $x_C = 12 \times n/M/1000 \times 100$ and $x_H = m/M/1000 \times 100$, substituting this into (7) yields the formula (1).

The regenerative organic substance preferably includes at least one selected from the group consisting of ethylene, propylene, solid carbon, ethane, propane, and phenol. Ethylene, propylene, solid carbon, ethane, propane, and phenol all generate a heating value Q of 4000 kJ/kg or more when converted to CO and $H_2$ by combustion reaction. Therefore, using these as the regenerative organic substance can more effectively reduce carbon dioxide emissions.

The regenerative organic substance is then introduced into the gas and reducing agent blowing device 4. The gas and reducing agent blowing device 4 is connected to the organic substance generation device 3 via the second dehydration device 6. The gas and reducing agent blowing device 4 has a reducing agent supply section that introduces the blown reducing agent into the tuyere 2 of the blast furnace 1 and a blast gas supply section that introduces the blast gas into the tuyere 2 of the blast furnace 1.

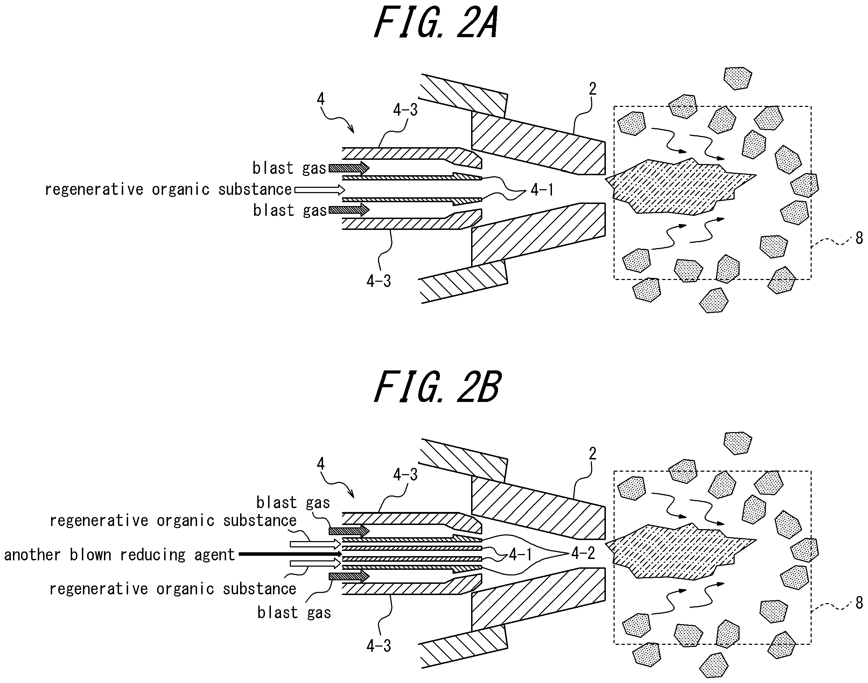

For example, as illustrated in FIG. 2A, the gas and reducing agent blowing device 4 has coaxial multiple pipes including a central pipe 4-1 and an outer pipe 4-3. Then, the regenerative organic substance is introduced into the inner passage of the central pipe, which serves as the reducing agent supply section (passage), and the blast gas is introduced into the annular passage between the central pipe 4-1 and the outer pipe 4-3, which serves as the blast gas supply section (passage).

The blowing amount of blown reducing agent should be controlled within a range that ensures the operational stability of the blast furnace and good combustibility at the tuyere outlet. For the stable operation of the blast furnace, it is preferable to control the tuyere-outlet temperature in the range of 2000 °C to 2400 °C. In order to ensure the combustibility of blown reducing agent at the tuyere outlet, the C/O ratio, expressed as (number of moles of C atoms in blown reducing agent) / (number of moles of O atoms in blast gas), is preferably 0.7 or less. Therefore, it is preferable to determine the blowing amount of blown reducing agent within a range such that tuyere-outlet temperature is 2000 °C or higher and 2400 °C or lower and the C/O ratio is 0.7 or less. No lower limit is particularly placed on the C/O ratio, but the C/O ratio may be preferably 0.1 or more.

Another blown reducing agent, for example, pulverized coal, waste plastics, and reducing gases such as hydrogen gas and carbon monoxide gas, may be used with the regenerative organic substance as long as the tuyere-outlet temperature is 2000 °C or higher and 2400 °C or lower and the C/O ratio is 0.7 or less. The total blowing amount of the another blown reducing agent other than the regenerative organic substance into the blast furnace is preferably 150 kg/t or less. Further, the total blowing amount of the another blown reducing agent other than the regenerative organic substance into the blast furnace is preferably more than 0 kg/t. Here, the unit of "kg/t" is the amount of the another blown reducing agent blown into the blast furnace in producing 1 t of hot metal.

When the another blown reducing agent is used, it may also be introduced into the regenerative organic substance supply section. When using pulverized coal or waste plastics as the another blown reducing agent, it is preferable to provide, separately from the regenerative organic substance supply section, another reducing agent supply section (passage) through which the pulverized coal or waste plastics flow. In this case, as illustrated in FIG. 2B, for example, the gas and reducing agent blowing device 4 has coaxial multiple pipes including, in addition to the central pipe 4-1 and outer pipe 4-3, an inner pipe 4-2 provided between the central pipe 4-1 and outer pipe 4-3. Then, the another blown reducing agent such as pulverized coal or waste plastics is introduced from the inner passage of the central pipe, which serves as the another reducing agent supply section, the regenerative organic substance is introduced from the annular passage between the central pipe 4-1 and the inner pipe 4-2, which is the regenerative organic substance gas supply section, and the blast gas is introduced from the annular passage between the inner pipe 4-2 and the outer pipe 4-3, which is the blast gas supply section.

Then, as illustrated in FIGS 2A and 2B, the blown reducing agent and blast gas introduced from the gas and reducing agent blowing device 4 are mixed in the tuyere 2. Immediately after this mixed gas is blown into the blast furnace 1 through the tuyere 2, rapid ignition and rapid combustion occur. Then, a raceway 8 that is an area where the oxygen gas reacts with the blown reducing agent such as blown methane gas and coke is formed in the blast furnace beyond the tuyere 2.

To further reduce carbon dioxide emissions, the sum of the mass ratio xc of carbon atoms in the regenerative organic substance and the mass ratio $x_H$ of hydrogen atoms in the regenerative organic substance, $x_C + x_H$ (hereinafter also referred to as $x_C + x_H$), is preferably 90 wt% or more. The carbon and hydrogen atoms undergo a combustion reaction at the tuyere, changing into CO and $H_2$ and reducing iron ore. On the other hand, elements other than the carbon and hydrogen atoms do not contribute to the reduction reaction of the iron ore. Therefore, it is preferable that no elements other than the carbon and hydrogen atoms be included in the regenerative organic substance. Thus, xc +$x_H$ is preferably 90 wt% or more, more preferably 95 wt% or more. No upper limit is particularly placed on $x_C + x_H$ and $x_C$

+ $x_H$ may be 100 wt%.

The mass ratio xc of carbon atoms in the regenerative organic substance is preferably 85 wt% or more. This is because as the ratio of carbon atoms in the regenerative organic substance is higher, the more C in the coke can be replaced and the amount of coke used can be reduced. No upper limit is particularly placed on the mass ratio xc of carbon atoms in the regenerative organic substance and xc may be 100 wt%. $x_C$ and $x_H$ are the ratios of the total mass of carbon atoms and hydrogen atoms, respectively, contained in the regenerative organic substance to the total mass of regenerative organic substance used as the blown reducing agent.

The regenerative organic substance preferably contains 90 wt% or more at least one of ethylene or propylene in total, that is, the total (mass) ratio of ethylene and propylene in the regenerative organic substance (hereinafter referred to as the total ratio of ethylene and propylene) is preferably 90 wt% or more. Ethylene and propylene are highly effective in reducing carbon dioxide emissions and are gaseous at room temperature, making them easy to handle and blow through the tuyere. The total ratio of ethylene and propylene is more preferably 95 wt% or more. The total ratio of ethylene and propylene is not particularly limited and may be 100 wt%.

In addition, the blown reducing agent preferably contains 80 wt% or more of regenerative organic substance. The ratio (mass ratio) of regenerative organic substance in the blown reducing agent is 80 wt% or more, thereby more effectively reducing carbon dioxide emissions. The ratio of regenerative organic substance in the blown reducing agent is preferably 90 wt% or more. No upper limit is particularly placed on the ratio of regenerative organic substance in the blown reducing agent and the ratio of regenerative organic substance may be 100 wt%.

To further reduce carbon dioxide emissions, it is preferable to increase the oxygen concentration in the blast gas. It is more preferable to use pure oxygen as the blast gas. By increasing the oxygen concentration in the blast gas, the decrease in tuyere-outlet temperature can be suppressed and the blowing amount of regenerative organic substance can be increased. Therefore, the effect of reducing carbon dioxide emissions rises further.

When pure oxygen is used as the blast gas, ignitability is worse than when hot blast (air heated to about 1200 °C) is used. Therefore, it is preferable to provide the discharge section of the outer pipe that constitutes the blast gas supply section of the gas and reducing agent blowing device 4 with a porous structure to promote the mixing of the oxygen gas and the blown reducing agent. When the oxygen concentration in the blast gas increases, the amount of gas in the furnace decreases, and the temperature rise of the burden at the upper part of the blast furnace may be insufficient. In this case, as illustrated in FIG. 4, it is preferable to perform preheated-gas blowing, in which part of the blast furnace by-product gas downstream of the first dehydration device 5 is partially combusted by a burner 10 to reach a temperature of about 800 °C to 1000 °C, and then blown into the blast furnace shaft section.

The regenerative organic substance may be generated from part of the blast furnace by-product gas and then, a surplus of the blast furnace by-product gas may be supplied inside or outside the steelworks. If there is a surplus of the regenerative organic gas, the surplus may be supplied inside or outside the steelworks.

Blast furnace operating conditions other than the above are not particularly limited, and conventional method should be followed. The method of producing hot metal according to one of the disclosed embodiments uses the above method of operating a blast furnace. Producing conditions of hot metal other than the above are not particularly limited and may be in accordance with conventional methods.

[Blast furnace ancillary facility]

The blast furnace ancillary facility (blast furnace ancillary apparatus) according to one of the disclosed embodiments is a blast furnace ancillary facility used in the method of operating a blast furnace as described above, comprising:

an organic substance generation device that uses the blast furnace by-product gas to generate the regenerative organic substance, and
a gas and reducing agent blowing device having a blown reducing agent supply section that introduces the blown reducing agent into the tuyere of the blast furnace and a blast gas supply section that introduces the blast gas into the tuyere of the blast furnace.

Here, the organic substance generation device has, for example, a blast furnace by-product gas intake section, a hydrogen gas intake section, and a reaction section. In the reaction section, the blast furnace by-product gas taken in from the blast furnace by-product gas intake section and the hydrogen gas taken in from the hydrogen gas intake section are reacted to produce the regenerative organic substance.

As described above, the gas and reducing agent blowing device has coaxial multiple pipes including the central pipe 4-1 and outer pipe 4-3, as illustrated in FIG. 2A, for example. Then, the regenerative organic substance that is a reducing agent is introduced into the inner passage of the central pipe, which serves as the reducing agent supply section (passage), and the blast gas is introduced into the annular passage between the central pipe 4-1 and the outer pipe 4-3, which serves as the blast gas supply section (passage).

Another blown reducing agent, for example, pulverized coal, waste plastics, or reducing gas such as hydrogen gas or carbon monoxide gas, may be used together.

When the another blown reducing agent is used, it may also be introduced into the reducing agent supply section along with the regenerative organic substance. When using pulverized coal or waste plastics as the another blown reducing agent, it is preferable to provide, separately from the reducing agent supply section for regenerative organic substance, another reducing agent supply section (passage) through which the pulverized coal or waste plastics flow. In this case, as illustrated in FIG. 2B, for example, the gas and reducing agent blowing device has coaxial multiple pipes including, in addition to the central pipe 4-1 and outer pipe 4-3, the inner pipe 4-2 provided between the central pipe 4-1 and outer pipe 4-3. Then, the another blown reducing agent such as pulverized coal or waste plastics is introduced from the inner passage of the central pipe, the regenerative organic substance is introduced from the annular passage between the central pipe 4-1 and the inner pipe 4-2, and the blast gas is introduced from the annular passage between the inner pipe 4-2 and the outer pipe 4-3, which is the blast gas supply section.

This disclosure enables further reduction of carbon dioxide ($CO_2$) emissions from the blast furnace to the outside under stable operation. The use of the regenerative organic substance generated from the blast furnace by-product gas can also reduce the amount of coke and pulverized coal used, that is, the amount of coal as a finite fossil fuel used.

EXAMPLES

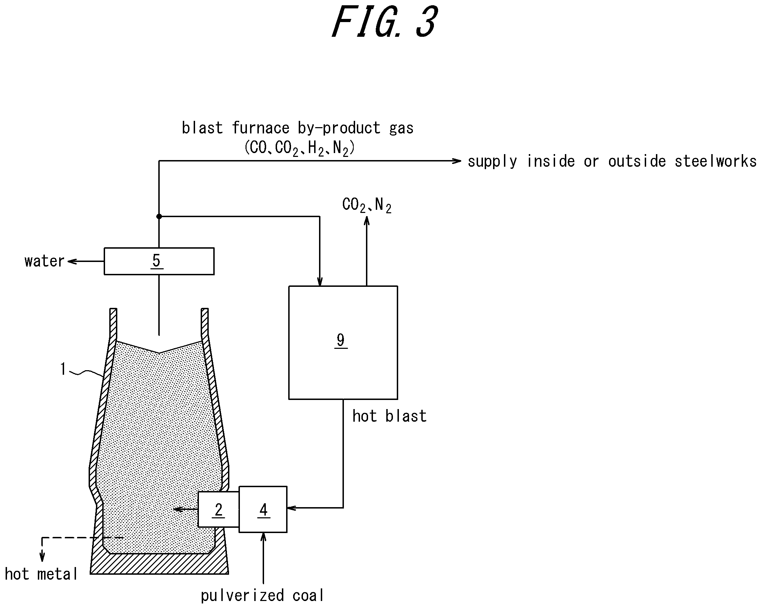

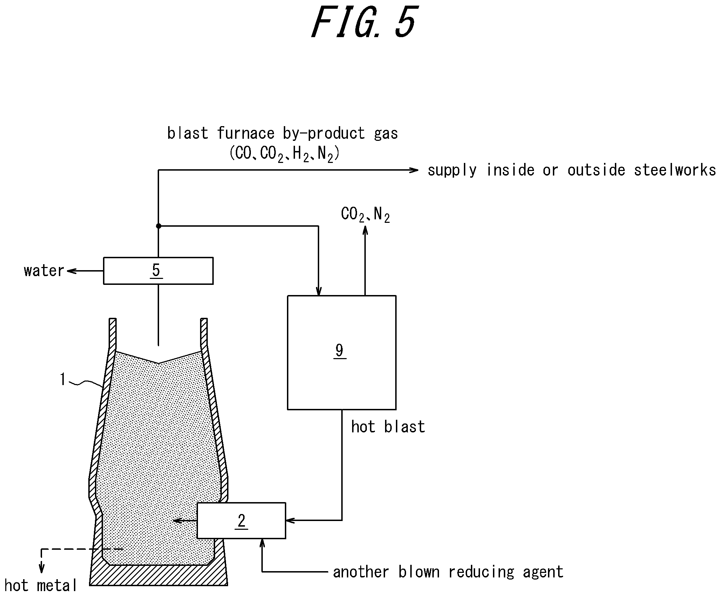

Using the blast furnaces and blast furnace ancillary facilities schematically illustrated in FIGS. 1 and 3 to 5, blast furnace operation was conducted under the conditions listed in Table 1, and the tuyere-outlet temperature and carbon dioxide emissions from the blast furnace during operation were evaluated. For Examples 1, 3 to 10 and Comparative Examples 2 and 3, the blast furnace and blast furnace ancillary facility schematically illustrated in FIG. 1 were used. For Comparative Example 2, the blast furnace and blast furnace ancillary facility schematically illustrated in FIG. 4 were used. For Comparative Example 1, the blast furnace and blast furnace ancillary facility schematically illustrated in FIG. 3 were used. For Comparative Example 4, the blast furnace and blast furnace ancillary facility schematically illustrated in FIG. 5 were used. The evaluation results are listed in Table 1.

[Table 1]

Table 1

| | | | Comp. Ex. 1 | Ex. 1 | Ex. 2 | Ex. 3 | Ex. 4 | Ex. 5 | Ex. 6 | Ex. 7 | Ex. 8 | Ex. 9 | Ex. 10 | Comp. Ex. 2 | Comp. Ex. 3 | Comp. Ex. 4 |
|---|---|---|---|---|---|---|---|---|---|---|---|---|---|---|---|---|
| Blast furnace specification | Shaft efficiency | - | 0.94 | 0.94 | 0.94 | 0.94 | 0.94 | 0.94 | 0.94 | 0.94 | 0.94 | 0.94 | 0.94 | 0.94 | 0.94 | 0.94 |
| | Heat loss | kcal/t | 100000.0 | 100000.0 | 100000.0 | 100000.0 | 100000.0 | 100000.0 | 100000.0 | 100000.0 | 100000.0 | 100000.0 | 100000.0 | 100000.0 | 1000000 | 100000.0 |
| | Coke rate | kg/t | 345 | 282 | 212 | 282 | 282 | 332 | 288 | 398 | 339 | 405 | 340 | 426 | 406 | 282 |
| | Reducing agent (brown reducing agent) — Pulverized coal rate | kg/t | 150 | 0 | 0 | 0 | 0 | 0 | 0 | 0 | 0 | 0 | 0 | 0 | 0 | 0 |
| | Reducing agent (brown reducing agent) — Regenerative organic substance rate | kg/t | 0 | 143 | 200 | 100 | 143 | 116 | 186 | 73 | 162 | 67 | 106 | 52 | 69 | 0 |
| | Reducing agent (brown reducing agent) — Another reducing agent (excl. pulverized coal) rate | kg/t | 0 | 0 | 0 | 43 | 0 | 0 | 0 | 0 | 0 | 0 | 0 | 0 | 0 | 143 |
| | Reducing agent (brown reducing agent) — Type of another reducing agent | - | - | - | - | - | $C_2H_4$ | - | - | - | - | - | - | - | - | $C_2H_4$ |
| | Ratio of regenerative organic substance in blown reducing agent | wt% | 0 | 100 | 100 | 70 | 100 | 100 | 100 | 100 | 100 | 100 | 100 | 100 | 100 | 0 |
| | Blast gas — Supply amount | Nm³/t | 1065 | 963 | 244 | 963 | 963 | 1026 | 1111 | 1085 | 1044 | 1059 | 1018 | 1098 | 1119 | 963 |
| | Blast gas — Supply temperature | °C | 1150 | 1150 | 25 | 1150 | 1150 | 1150 | 1150 | 1150 | 1150 | 1150 | 1150 | 1150 | 1150 | 1150 |
| | Blast gas — Type | - | hot blast | hot blast | oxygen gas | hot blast | hot blast | hot blast | hot blast | hot blast | hot blast | hot blast | hot blast | hot blast | hot blast | hot blast |
| | Blast gas — Oxygen concentration | vol.% | 23 | 23 | 100 | 23 | 23 | 23 | 23 | 23 | 23 | 23 | 23 | 23 | 23 | 23 |
| | Generation amount of blast furnace by-product gas | Nm³/t | 1569 | 1474 | 740 | 1474 | 1474 | 1539 | 1596 | 1601 | 1577 | 1603 | 1532 | 1615 | 1651 | 1474 |

(continued)

| Gas separation process | | Unit | Comp. Ex. 1 | Ex. 1 | Ex. 2 | Ex. 3 | Ex. 4 | Ex. 5 | Ex. 6 | Ex. 7 | Ex. 8 | Ex. 9 | Ex. 10 | Comp. Ex. 2 | Comp. Ex. 3 | Comp. Ex. 4 |
|---|---|---|---|---|---|---|---|---|---|---|---|---|---|---|---|---|
| | Presence/absence of separation process | - | absent | present | absent | present | present | present | present | present | present | present | present | present | present | absent |
| | Gas type used in separation process | - | - | blastfurnace by-product gas | - | blastfurnace by-product gas | blast furnace by-product gas | blast furnace by-product gas | blast furnace by-product gas | blastfurnace by-product gas | blast furnace by-product gas | blast furnace by-product gas | blastfurnace by-product gas | blast furnace by-product gas | blastfurnace by-product gas | - |
| | Gas amount used in separation process | $Nm^3/t$ | - | 571 | - | 399 | 1142 | 455 | 770 | 270 | 533 | 248 | 407 | 175 | 243 | - |
| | Gas type after separation | - | - | $CO, CO_2$ | - | $CO, CO_2$ | $CO, CO_2$ | $CO, CO_2$ | $CO, CO_2$ | $CO, CO_2$ | $CO, CO_2$ | $CO, CO_2$ | $CO, CO_2$ | $CO, CO_2$ | $CO, CO_2$ | - |
| | Gas amount after separation | $Nm^3/t$ | - | 283 | - | 198 | 458 | 186 | 347 | 111 | 232 | 102 | 198 | 72 | 116 | - |

Table 1 (cont'd)

| | | | Comp. Ex. 1 | Ex. 1 | Ex. 2 | Ex. 3 | Ex. 4 | Ex. 5 | Ex. 6 | Ex. 7 | Ex. 8 | Ex. 9 | Ex. 10 | Comp. Ex. 2 | Comp. Ex. 3 | Comp. Ex. 4 |
|---|---|---|---|---|---|---|---|---|---|---|---|---|---|---|---|---|
| Regenerative organic substance generation process | Raw material gas type | - | - | $CO, CO_2$ | $CO, CO_2, H_2$ | $CO, CO_2$ | $CO, CO_2$ | $CO, CO_2$ | $CO, CO_2$ | $CO, CO_2$ | $CO, CO_2$ | $CO, CO_2$ | $CO, CO_2$ | $CO, CO_2$ | $CO, CO_2$ | - |
| | Raw material gas amount | $Nm^3/t$ | - | 283 | 416 | 198 | 458 | 186 | 347 | 111 | 232 | 102 | 198 | 72 | 116 | - |
| | Hydrogen gas supply amount | $Nm^3/t$ | - | 526 | 726 | 368 | 1158 | 468 | 522 | 317 | 427 | 290 | 384 | 242 | 321 | - |
| | Generation amount of regenerative organic substance | kg/t | - | 143 | 200 | 100 | 286 | 116 | 186 | 73 | 162 | 67 | 106 | 52 | 69 | - |
| | Type of regenerative organic substance | - | - | $C_2H_4$ | $C_2H_4$ | $C_2H_4$ | $C_2H_4$ | $C_3H_6$ | C | $C_3H_8$ | phenol | $C_2H_6$ | $C_2H_4:CH_4 =8:2$ (mass ratio) | $CH_4$ | $CH_4$ | - |
| | Composition of regenerative organic substance — $X_C$ | wt% | - | 85.7 | 85.7 | 85.7 | 85.7 | 85.7 | 100.0 | 81.8 | 76.6 | 80.0 | 83.6 | 75.0 | 75.0 | - |
| | Composition of regenerative organic substance — $X_H$ | wt% | - | 14.3 | 14.3 | 14.3 | 14.3 | 14.3 | 0.0 | 18.2 | 6.4 | 20.0 | 16.4 | 25.0 | 25.0 | - |
| | Composition of regenerative organic substance — $X_C+X_H$ | wt% | - | 100.0 | 100.0 | 100.0 | 100.0 | 100.0 | 100.0 | 100.0 | 83.0 | 100.0 | 100.0 | 100.0 | 100.0 | - |
| | Composition of regenerative organic substance — others | wt% | - | 0.0 | 0.0 | 0.0 | 0.0 | 0.0 | 0.0 | 0.0 | 17.0 | 0.0 | 0.0 | 0.0 | 0.0 | - |
| | Heating value Q of regenerative organic substance | kJ/kg | - | 9763 | 9763 | 9763 | 9763 | 8371 | 9210 | 5156 | 6029 | 4575 | 8256 | <u>2228</u> | <u>2228</u> | - |
| | Total ratio of ethylene and propylene | wt% | - | 100 | 100 | 100 | 100 | 100 | 0 | 0 | 0 | 0 | 80 | 0 | 0 | - |

(continued)

| | | | Comp. Ex. 1 | Ex. 1 | Ex. 2 | Ex. 3 | Ex. 4 | Ex. 5 | Ex. 6 | Ex. 7 | Ex. 8 | Ex. 9 | Ex. 10 | Comp. Ex. 2 | Comp. Ex. 3 | Comp. Ex. 4 |
|---|---|---|---|---|---|---|---|---|---|---|---|---|---|---|---|---|
| Organic substance and blast furnace by-product gas distribution | Regenerative organic substance used as blown reducing agent | kg/t | - | 143 | 200 | 100 | 143 | 116 | 186 | 73 | 162 | 67 | 106 | 52 | 69 | - |
| | Regenerative organic substance supplied inside or outside steelworks | kg/t | - | 0 | 0 | 0 | 143 | 0 | 0 | 0 | 0 | 0 | 0 | 0 | 0 | - |
| | Surplus amount of blast furnace by-product gas (supply amount inside steelworks) | $Nm^3/t$ | 1569 | 903 | 324 | 1075 | 332 | 1084 | 826 | 1331 | 1044 | 1355 | 1125 | 1440 | 1408 | 1474 |
| C balance | Consumption rate of circulating carbon atoms | kg/t | 0 | 123 | 171 | 86 | 123 | 99 | 186 | 60 | 124 | 54 | 89 | 39 | 52 | 0 |
| | Blast furnace Input C | kg/t | 415 | 243 | 182 | 279 | 243 | 236 | 248 | 342 | 292 | 348 | 309 | 366 | 349 | 365 |
| Evaluation results | Amount of $CO_2$ emitted from blast furnace to outside | kg/t | 1523 | 889 | 668 | 1025 | 889 | 1045 | 908 | 1254 | 1069 | 1277 | 1132 | 1342 | 1279 | 1340 |
| | Tuyere-outlet temperature | °C | 2099 | 2000 | 2397 | 2000 | 2000 | 2000 | 2138 | 2000 | 2000 | 2000 | 2000 | 2000 | 1900 | 2000 |
| | C/O ratio | - | 0.45 | 0.52 | 0.66 | 0.52 | 0.52 | 0.39 | 0.68 | 0.22 | 0.48 | 0.20 | 0.35 | 0.14 | 0.19 | 0.52 |

In the examples, $CO_2$ emissions were reduced to 1300 kg/t or less. The tuyere-outlet temperature was 2000 °C or higher and stable operation was possible. In contrast, in the comparative examples, $CO_2$ emissions exceeded 1300 kg/t or the tuyere-outlet temperature was lower than 2000 °C, indicating problems with $CO_2$ emissions or operational stability.

REFERENCE SIGNS LIST

1: blast furnace
2: tuyere
3: organic substance generation device
4: gas and reducing agent blowing device
4-1: central pipe
4-2: inner pipe
4-3: outer pipe
5: first dehydration device
6: second dehydration device
7: gas separation device
8: raceway
9: hot air oven
10: burner

**Claims**

1.  A method of operating a blast furnace, comprising

    blowing a blast gas and a blown reducing agent into the blast furnace through a tuyere,
    wherein the blown reducing agent contains a regenerative organic substance,
    the regenerative organic substance is generated using at least one of carbon monoxide or carbon dioxide in a blast furnace by-product gas, which is a by-product gas discharged from the blast furnace, with hydrogen, and
    the regenerative organic substance has a heating value Q of 4000 kJ/kg or more when converted to CO and $H_2$ by combustion reaction.

2.  The method of operating a blast furnace according to claim 1, wherein $x_C + x_H$, a sum of a mass ratio xc of carbon atoms in the regenerative organic substance and a mass ratio $x_H$ of hydrogen atoms in the regenerative organic substance, is 90 wt% or more.

3.  The method of operating a blast furnace according to claim 1 or 2, wherein the mass ratio xc of carbon atoms in the regenerative organic substance is 85 wt% or more.

4.  The method of operating a blast furnace according to any one of claims 1 to 3, wherein the regenerative organic substance contains at least one selected from the group consisting of ethylene, propylene, solid carbon, ethane, propane, and phenol.

5.  The method of operating a blast furnace according to any one of claims 1 to 4, wherein the regenerative organic substance contains 90 wt% or more in total of at least one of ethylene or propylene.

6.  The method of operating a blast furnace according to any one of claims 1 to 5, wherein the blown reducing agent contains 80 wt% or more of the regenerative organic substance.

7.  The method of operating a blast furnace according to any one of claims 1 to 6, wherein the regenerative organic substance is generated from part of the blast furnace by-product gas and a surplus of the blast furnace by-product gas is supplied inside or outside a steelworks.

8.  The method of operating a blast furnace according to any one of claims 1 to 7, wherein a surplus of the regenerative organic substance is supplied inside or outside the steelworks.

9. A method of producing hot metal using the method of operating a blast furnace according to any one of claims 1 to 8.

10. A blast furnace ancillary facility used in the method of operating a blast furnace according to any one of claims 1 to 8, comprising:
an organic substance generation device that uses the blast furnace by-product gas to generate the regenerative organic substance, and
a gas and reducing agent blowing device having a blown reducing agent supply section that introduces the blown reducing agent into the tuyere of the blast furnace and a blast gas supply section that introduces the blast gas into the tuyere of the blast furnace.

# FIG. 1

blast furnace by-product gas
($CO$, $CO_2$, $H_2$, $N_2$)     supply inside or outside steelworks →

$CO_2$, $N_2$ ↑

water ← | 5 |

| 7 | → $N_2$, $H_2$

$CO/CO_2$

| 9 |

| 3 | ← $H_2$

hot blast

regenerative organic
substance + water

| 6 | → water

1

regenerative organic substance

| 2 | 4 |

→ ↑

hot metal

regenerative
organic substance

supply inside or
outside steelworks →

another blown reducing agent

FIG. 2A

FIG. 2B

# FIG. 3

blast furnace by-product gas
$(CO、CO_2、H_2、N_2)$ → supply inside or outside steelworks

$CO_2、N_2$

water ← [5]

[9]

1

[2] [4]

hot blast

hot metal

pulverized coal

# FIG. 4

blast furnace by-product gas
$(CO, CO_2, H_2, N_2)$        supply inside or outside steelworks

water ←  [ __5__ ]

preheated-gas
blowing

[ __3__ ] ← $H_2$

regenerative organic
substance + water

[ __6__ ] → water

1

[ __10__ ]

regenerative organic substance

regenerative
organic substance

supply inside or outside steelworks

[ __2__ ] [ __4__ ] ← oxygen

hot metal

another blown reducing agent

# FIG. 5

blast furnace by-product gas
($CO$、$CO_2$、$H_2$、$N_2$)

supply inside or outside steelworks

$CO_2$、$N_2$

water

5

9

hot blast

1

2

hot metal

another blown reducing agent

## INTERNATIONAL SEARCH REPORT

International application No.

**PCT/JP2022/040553**

### A.    CLASSIFICATION OF SUBJECT MATTER

*C21B 5/00*(2006.01)i; *C07C 29/151*(2006.01)i; *C21B 5/06*(2006.01)i; *C21B 7/00*(2006.01)i; *F27D 17/00*(2006.01)i
FI:   C21B5/00 321; C07C29/151; C21B5/00 320; C21B5/06; C21B7/00 307; C21B7/00 308; C21B7/00 312; F27D17/00
104G

According to International Patent Classification (IPC) or to both national classification and IPC

### B.    FIELDS SEARCHED

Minimum documentation searched (classification system followed by classification symbols)

C21B5/00; C07C29/151; C21B5/06; C21B7/00; F27D17/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C.    DOCUMENTS CONSIDERED TO BE RELEVANT

| Category* | Citation of document, with indication, where appropriate, of the relevant passages | Relevant to claim No. |
|---|---|---|
| Y | JP 2006-207009 A (JFE STEEL KK) 10 August 2006 (2006-08-10)<br>claims 1-3, paragraphs [0008]-[0027], fig. 1-3 | 1-10 |
| Y | JP 2011-084528 A (MITSUBISHI CHEMICALS CORP.) 28 April 2011 (2011-04-28)<br>paragraphs [0001], [0007], [0019]-[0084], fig. 1 | 1-10 |
| Y | JP 63-171804 A (NKK CORP.) 15 July 1988 (1988-07-15)<br>claims 1, 2, page 1, lower left column, line 17 to page 2, upper left column, line 19, page 2, lower right column, line 15 to page 3, lower left column, line 2, drawings | 7-9 |
| A | JP 2009-235482 A (JFE STEEL KK) 15 October 2009 (2009-10-15)<br>entire text | 1-10 |
| A | JP 2002-371307 A (NKK CORP.) 26 December 2002 (2002-12-26)<br>entire text | 1-10 |

☐ Further documents are listed in the continuation of Box C.     ☑ See patent family annex.

| | |
|---|---|
| *     Special categories of cited documents:<br>"A"   document defining the general state of the art which is not considered to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| **18 November 2022** | **06 December 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2022/040553**

| Patent document cited in search report | | | Publication date (day/month/year) | Patent family member(s) | Publication date (day/month/year) |
|---|---|---|---|---|---|
| JP | 2006-207009 | A | 10 August 2006 | (Family: none) | |
| JP | 2011-084528 | A | 28 April 2011 | (Family: none) | |
| JP | 63-171804 | A | 15 July 1988 | (Family: none) | |
| JP | 2009-235482 | A | 15 October 2009 | (Family: none) | |
| JP | 2002-371307 | A | 26 December 2002 | (Family: none) | |

Form PCT/ISA/210 (patent family annex) (January 2015)

**REFERENCES CITED IN THE DESCRIPTION**

**Patent documents cited in the description**

- JP 2011225969 A

- JP 2014005510 A